(19) Europäisches Patentamt
European Patent Office
Office européen des brevets

(11) EP 1 390 027 B1

(12) EUROPEAN PATENT SPECIFICATION

(45) Date of publication and mention of the grant of the patent:
**02.04.2008 Bulletin 2008/14**

(21) Application number: **01951327.4**

(22) Date of filing: **31.05.2001**

(51) Int Cl.:
***A61K 31/365*** *(2006.01)* ***A61K 31/343*** *(2006.01)*
***A61K 31/555*** *(2006.01)* ***A61P 35/00*** *(2006.01)*

(86) International application number:
**PCT/CN2001/000885**

(87) International publication number:
**WO 2002/096413 (05.12.2002 Gazette 2002/49)**

(54) **COMPOSITION COMPRISING DEMETHYLCANTHARIDIN IN COMBINATION WITH PLATINUM-CONTAINING ANTICANCER AGENTS AND USE THEREOF**

ZUSAMMENSETZUNG MIT DEMETHYLCANTHARIDIN IN KOMBINATION MIT PLATINHALTIGEN ANTIKREBSMITTELN UND IHRE VERWENDUNG

COMPOSITION COMPRENANT UNE DEMETHYLCANTHARIDINE EN COMBINAISON AVEC DES AGENTS ANTICANCEREUX CONTENANT DU PLATINE ET SON UTILISATION

(84) Designated Contracting States:
**AT BE CH CY DE DK ES FI FR GB GR IE IT LI LU MC NL PT SE TR**

(43) Date of publication of application:
**25.02.2004 Bulletin 2004/09**

(73) Proprietor: **THE CHINESE UNIVERSITY OF HONG KONG**
**Hong Kong SAR (CN)**

(72) Inventors:
- **AU-YEUNG, Steve C.F.**
  **Yuen Long,**
  **N.T.,**
  **Hong Kong SAR (CN)**
- **TO, Kenneth K.W.**
  **Lokf u,**
  **Kowloon,**
  **Hong Kong SAR (CN)**
- **HO, Yee-Ping**
  **Fo Tan,**
  **N.T. Hong Kong SAR (GB)**

(74) Representative: **Krauss, Jan**
**Forrester & Boehmert**
**Pettenkoferstrasse 20-22**
**80336 München (DE)**

(56) References cited:
**WO-A1-00/04023** **US-A- 6 110 907**

- **ER-BIN YANG ET ALL.: "Norcantharidin inhibits growth of human HepG2 cell-transplanted tumor in nude mice and prolongs host survival" CANCER LETTERS, vol. 117, 1993, pages 93-98, XP002278952**
- **JING JIE YU ET ALL.: "Preliminary study of the effect of selected Chinese natural drugs on human ovarian cancer cells" ONCOLOGY REPORTS, vol. 2, 1995, pages 571-575, XP008030375**
- **DATABASE CAPLUS [Online] DN 100:17293, 1984 WANG GUANGSHENG: 'Hydrolysis and demethylation of cantharidin of the relief of its urinary', XP002969380 Database accession no. 1984:17293 & YAOXUE TONGBAO vol. 18, no. 7, 1983, pages 402 - 403**
- **DATABASE CAPLUS [Online] DN 95:197010, 1981 YUNG KUOHUANG ET AL.: 'Preliminary observation on using CaEs-17 cell line for in vitro', XP002969379 Database accession no. 1981:597010 & J. OF CHINESE TUMOR vol. 3, no. 1, 1981, page 77**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 390 027 B1

## Description

### TECHNICAL FIELD OF THE INVENTION

The invention relates to a pharmaceutical composition for inhibiting tumorous cells comprising demethylcantharidin, in particular, to a pharmaceutical composition for inhibiting cisplatin, carboplatin or oxaliplatin-sensitive and resistant tumorous cells comprising a combination of demethycantharidin with a platinum anticancer agent, and a use thereof.

### BACKGROUND OF THE INVENTION

Demethylcantharidin (DMC) is a modified structure of cantharidin, which is an active component of "blister beetles". Blister beetles have been used as a traditional Chinese medicine (TCM) to treat liver, lung and digestive tract tumors.

Cisplatin and carboplatin, both being platinum (Pt)-base drugs, have clinically been used for the treatment of cancers worldwide. Common problems associated with these drugs include toxic side effects and drug resistance. However, a recent invention (Au-Yeung et al, US Patent 6,110,907. has shown that demethylcantharidin platinum complex with structure I or II (see Au-Yeung's US Patent 6,110,907) demonstrates *in vitro* antitumor activity. The inventors' latest studies have shown that the platinum-containing complex in the patent is of in vivo good activity, circumvention of cisplatin resistance and selectivity towards breast and liver cancers. The latter two properties (circumvention of resistance and selectivity) are attributed to the inclusion of DMC.

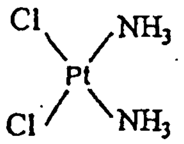

Cisplatin

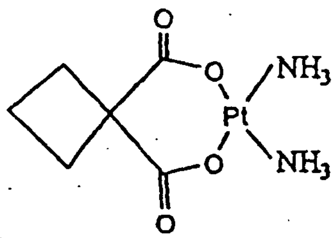

Carboplatin

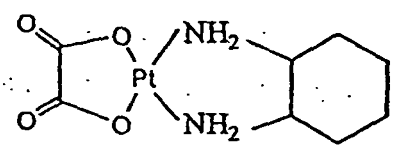

Oxaliplatin

Demethylcantharidin Platinum Complexes Disclosed in USP6,110,967

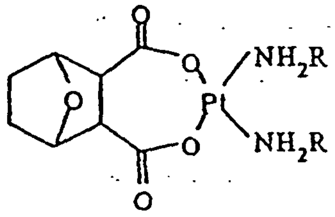

Structure I

R = H, C1-C10 or ring size C3-C6;

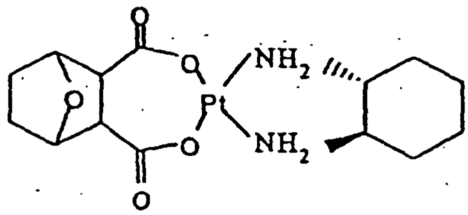

Structure II

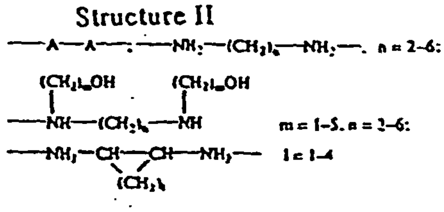

Er-Bin Yang et al. (Cancer Letters 117: 93-98, 1997) disclose the use of DMC for the treatment of human hepatocellular carcinoma HepG2 cells.

WO 00/04023 A1 discloses anhydride modified cantharidin analogues, such as DMC, useful in the treatment of certain forms of cancer. Also the use of DMC in conjunction with one or more further treatments for treating cancer is disclosed, wherein the one or more cancer treatments can be a treatment involving the platinum compound cisplatin.

The inventors have surprisingly found that DMC or its hydrolyzed form has synergistic effect in inhibiting the growth of cisplatin, carboplatin or oxaliplatin-sensitive and -resistant tumorous cells in combination with a Pt-containing anticancer agent. The present invention is hereby provided.

## Summary of the Invention

The invention provides a pharmaceutical composition comprising a combination of demethylcantharidin (DMC) or its hydrolyzed form with a platinum anticancer agent having a synergistic effect in inhibiting the growth of cisplatin, carboplatin or oxaliplatin-sensitive and -resistant tumorous cells, wherein a molar ratio between DMC as its hydrolyzed form and the platinum anticancer agent is from 55:1 to 1:100.

The invention provides a use of a combination of demethylcantharidin (DMC) or its hydrolyzed from with a platinum anticances agent in manufacturing a medicament for inhibiting the growth of cisplatin, carboplatin or oxaliplatin-sensitive and -resistant tumorous cells in a subject, wherein a molar ratio between DMC as its hydrolyzed form and the platinum anticancer agent is from 55:1 to 1:100.

## BRIEF DESCRIPTION OF THE DRAWINGS

Fig.1 shows representative isobologram for cisplatin plus DMC in sensitive L1210 mouse leukaemia.

Fig. 2 shows representative isobologram for cisplatin plus DMC in cisplatin-resistant L1210 mouse leukaemia.

Fig. 3 shows representative isobologram for carboplatin plus DMC in sensitive L1210 mouse leukaermia.

Fig. 4 shows representative isobologram for carboplatin plus DMC in cisplatin-resistant L1210 mouse leukaemia.

Fig. 5 shows representative isobologram for oxaliplatin plus DMC in sensitive L1210 mouse leukaemia.

Fig. 6 shows representative isobologram for oxaliplatin plus DMC in cisplatin-resistant L1210 mouse leukaemia.

Fig. 7 shows representative isobologram for complex 1 plus DMC in sensitive L1210 mouse leukaemia.

Fig. 8 shows representative isobologram for complex 1 plus DMC in cisplatin-resistant L1210 mouse leukaemia.

Fig. 9 shows representative isobologram for complex 5 plus DMC in sensitive L1210 mouse leukaemia.

Fig. 10 shows representative isobologram for complex 5 plus DMC in cisplatin-resistant L1210 mouse leukaemia.

Fig. 11. Shows Fa-CI plot diagram for the combination of cisplatin with DMC (1:4.5) in cisplatin-resistant L1210.

DETAILED DESCRIPTION OF THE INVENTION

A "hydrolyzed form" used herein means any pharmaceutically acceptable forms obtained by hydrolysis of DMC or derivative thereof including diacid, mono acid, ester, salt. In one embodiment, the hydrolyzed form is diacid of DMC.

The tumorous cells mentioned above are derived from lung, liver, breast, colon, ovary, cervix, leukemia, lymphoma, or naso-pharyngeal carcinoma.

An "effective amount" is any amount effective to inhibit the growth of the tumorous cells in a subject which in general involves animals or human being. Methods of determining an effective amount is well known to those skilled in the art and depends on the factors including, the type of subject involved, the size if the blood sample contacted and the detectable marker used.

The term "administering" used in the invention means any of standard administration known to those skilled in the art. Examples include, intravenous, intraperitoneal or intramuscular administration.

The invention provides a pharmaceutical composition comprising a combination of demethylcantharidin or its hydrolyzed form with a platinum anticancer agent having a synergistic effect in inhibiting the growth of cisplatin, carboplatin or oxaliplatin-sensitive and -resistant tumorous cells and a pharmaceutically, acceptable carrier. In the composition, the molar ratio between DMC or its hydrolyzed form and platinum anticancer agents is from 55:1 to 1:100, preferably from 10:1 to 1:10, and most preferably 1:1 (ratios referred to hereinafter meaning molar ratios except a specific indication). Obviously, the pharmaceutical composition according to the invention may comprise one or more platinum anticancer agents.

The purpose of combining two or more therapeutic agents is to achieve lower drug doses, reduce toxicity and minimize or delay the emergence of resistance by target cells, and to provide a potential synergistic effect. This is the first report of a synergistic combination of demethylcantharidin with a platinum-containing anticancer agent demonstrating antitumor activity in cisplatin, carboplatin or oxaliplatin-sensitive and -resistant cell lines. Combinations of different Pt-contaihirig anticancer agents with DMC, in varying ratios are potential new candidates for chemotherapeutic explorations

The platinum anticancer agent used in the invention includes any platinum-containing agents having been used or to be used in treating cancers. In the invention, the platinum anticancer agent is preferably selected from cisplatin, carboplatin, oxaliplatin, and all complexes claimed in USP6,110,907. Of the complexes in USP6,110,907, complexes 1 to 5 are preferable, which have the following structures:

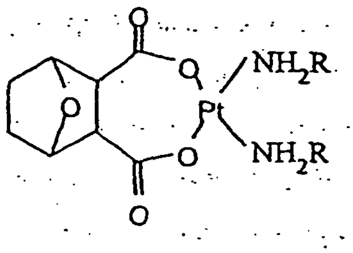

Complexes 1-4

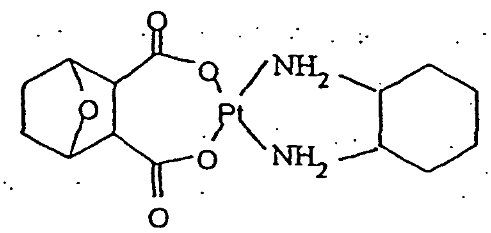

Complex 5

(in complexes 1-4, R being H, $CH(CH_3)_2$, $CH_3$, $C_2H_5$, respectively)

The invention provides a use of demethylcantharidin (DMC) or its hydrolyzed form in manufacturing a medicament for inhibiting the growth of cisplatin, carboplatin or oxaliplatin-sensitive and -resistant tumorous cells comprising combining DMC or its hydrolyzed form with a platinum anticancer agent as well as a pharmaceutically acceptable carrier wherein a molar ratio between DMC or its hydrolysed form and the platinum anticancer agent is from 55:1 to 1:100. Methods in combination of DMC or its hydrolyzed form with the platinum anticancer agent and the pharmaceutically acceptable carrier are well known to those skilled in the art. Pharmaceutical acceptable carriers are also well known to those skilled in the art and may be aqueous or non-aqueous solutions, suspensions, emulsions and solid forms. Examples of the carrier are propylene glycol, polyethylene glycol, vegetable oils such as olive oil, water, saline, dextrose, $CaCO_3$ and powders of corn. The tumorous cells are derived from lung, liver, breast, colon, ovary, cervix, leukemia, lymphoma, or naso-pharyngeal carcinoma.

As single agents, DMC or its hydrolyzed form, and Pt-containing complexes of Structures I and II disclosed in USP6,110,907 demonstrate good *in vitro* antitumor activity in a variety of cell lines as shown in Table 1 and Table 2.

**Table 1: In vitro antitumor activity IC50 (μM) of DMC, its diacid and Cantharidin**

| | $IC_{50}$ (μM) | | |
|---|---|---|---|
| Compound | DMC | Hydrolyzed DMC | Cantharidin |
| Cell | | | |
| L1210 | 13.9 | 8.327 | 38.9 |
| COLO320DM | 19.27 | 46.48 | 35.64 |
| MDA-MB-231 | 31.791 | 41.725 | 10.622 |
| SK-OV-3 | 53.799 | 46.620 | 4.978 |
| SK-Hep-1 | 11.475 | 7.203 | 3.338 |

**Table 2. Growth-inhibitory activity ($IC_{50}$/μM ± sd; n = 12-16 from 3-4 independent experiments) of DMC and Complexes 1-5**

| Cell Line | Cisplatin | Carboplatin | 1 | 2 | 3 | 4 | 5 |
|---|---|---|---|---|---|---|---|
| **L1210 mouse leukaemia** | 0.51 ± 0.11 | 10.14 ± 3.71 | 2.71 ± 0.40 | 12.08 ± 2.35 | 6.19 ± 2.67 | 5.92 ± 1.94 | 0.12 ± 0.05 |
| **COL0320-DM human colon cancer** | 11.90 ± 1.50 | 188.03 ± 35.75 | 37.25 ± 7.14 | 84.68 ± 9.68 | 48.92 ± 10.51 | 48.46 ± 9.95 | 10.10 ± 4.38 |
| **SK-Hep-1 human liver cancer** | 54.15 ± 9.81 | 500.56 ± 86.57 | 20.59 ± 5.89 | 17.18 + 8.07 | 15.44 ± 6.04 | 19.99 ± 5.52 | 2.97 ± 0.45 |
| **MDA-MB-231 human breast cancer** | 65.74 ± 4.39 | 1082.66 ± 156.81 | 63.80 ± 8.28 | 94.45 ± 9.61 | 49.43 ± 9.42 | 62.53 ± 13.32 | 36.53 ± 13.81 |
| **NCI:H460 human lung cancer** | 11.32 ± 4.93 | 60.26 ± 16.37 | 21.14 ± 10.65 | 26.46 ± 8.09 | 3.44 ± 1.98 | 3.91 ± 2.65 | 3.14 ± 0.25 |
| **SK-OV-3 human ovarian cancer** | 15.16 ± 11.75 | 148.73 ± 34.13 | 40.75 ± 14.27 | 77.65 ± 19.81 | 37.19 ± 13.39 | 62.65 ± 33.51 | 41.93 + 14.96 |
| **NTERA-S cl D1 human testicular cancer** | 0.19 ± 0.10 | 2.75 ± 1.13 | 0.36 ± 0.25 | 1.44 ± 0.41 | 0.84 ± 0.20 | 0.60 ± 0.24 | 0.15 ± 0.09 |

**Synergistic effect of combinations of platinum-containing anticancer drugs with demethylcantharidin (DMC) or its hydrolyzed form**

Example 1: Cisplatin in combination with DMC

The combination of platinum anticancer drugs with demethylcantharidin or its hydrolyzed form (the diacid), in different proportions, show a synergistic effect in both, sensitive and cisplatin-resistant tumorous cells. For example, as a result of synergy, the dose of cisplatin needed to achieve an antitumor effect (equivalent to cisplatin alone), may be

reduced by twofold when given in combination with demethylcantharidin (in a molar ratio of not exceeding 1:10), in a sensitive L1210 cell line (Table 3). For comparable ratios of cisplatin and DMC, this dose reduction is substantially more dramatic in a cisplatin-resistant L1210 cell line (40 fold resistant to cisplatin). That is, the dose of cisplatin needed to achieve an equivalent antitumor effect is reduced by sixfold (Cisplatin:DMC at 1:1, molar ratio) to 27-fold (Cisplatin:DMC at 1:6, molar ratio) (Table 4).

From Tables 3 and 4, the dose reduction indices (DRI) for cisplatin in cisplatin-resistant L1210 were generally greater than that in cisplatin-sensitive L1210 cell lines. This can explain why combining DMC with cisplatin can potentially overcome cisplatin resistance by the synergism (Figs 1 and 2) between cisplatin and DMC.

Dose-effect relationships of cisplatin and demethylcantharidin (DMC) and their combinations on growth inhibition of sensitive and cisplatin-resistant L1210 mouse leukaemia cell line during 72-hr exposure*

**Table 3: Sensitive parental L1210 mouse leukaemia**

| | Parameters at fa = 50% | | | | |
|---|---|---|---|---|---|
| Compounds | Dm | m | r | CI at fa = 50% | DRI# at fa = 50% |
| Cisplatin | 0.6031 | 1.2405 | 0.9884 | | |
| Demethylcantharidin (DMC) | 11.1853 | 2.4154 | 0.9852 | | |
| Cisplatin + DMC (3.36:1) | 0.6899 | 1.0312 | 0.9923 | 0.8958 | 1.13, 70.70 |
| Cisplatin + DMC (1.68:1) | 0.8361 | 1.0254 | 0.9954 | 0.8969 | 1.15,35.86 |
| **Cisplatin + DMC (1:1.49)** | 1.3018 | 1.3556 | 0.9849 | 0.9365 | **1.15,14.36** |
| Cisplatin + DMC (1:3.57) | 2.2259 | 1.3635 | 0.9968 | 0.9630 | 1.24, 6.43 |
| **Cisplatin + DMC (1:6.70)** | 3.1458 | 1.6396 | 0.9689 | 0.9224 | **1.48, 4.09** |
| Cisplatin + DMC (1:11.91) | 4.7006 | 1.8092 | 0.9984 | 0.9916 | 1.66,2.58 |
| Cisplatin + DMC (1:22.32) | 5.2656 | 1.3586 | 0.9258 | 0.8250 | 2.67,2.22 |
| Cisplatin + DMC (1:53:57) | 4.4805 | 1.2625 | 0.9894 | 0.5294 | 7.35,2.54 |
| All CI values ≤ 1, indicating synergistic effects between cisplatin & DMC | | | | | |

**Table 4: Cisplatin-resistant L1210 mouse leukaemia** (-40-fold resistant to cisplatin)

| | Parameters at fa = 50% | | | | |
|---|---|---|---|---|---|
| Compounds | Dm | m | r | CI at fa = 50% | DRI# at fa = 50% |
| Cisplatin | 24.6021 | 1.6239 | 0.9958 | | |
| Demethylcantharidin (DMC) | 11.9690 | 1.2116 | 0.9807 | | |
| Cisplatin + DMC (6.72:1) | 20.9452 | 1.7906 | 0.9999 | 0.9678 | 1.34,4.41 |
| Cisplatin + DMC (2.69:1) | 17.4654 | 1.3607 | 1.0000 | 0.9131 | 1.93,2.53 |
| Cisplatin + DMC (1.12:1) | 13.2510 | 1.4904 | 0.9956 | 0.8068 | 3.51,1.91 |
| **Cisplatin + DMC (1:1.67)** | 10.2430 | 1.7571 | 0.9927 | 0.6915 | **6.42, 1.87** |
| Cisplatin + DMC (1:2.98) | 8.9790 | 1.4650 | 0.9938 | 0.6533 | 10.89, 1.78 |
| **Cisplatin + DMC (1:5.58)** | 6.0791 1 | 1.5703 | 0.9989 | 0.4683.. | **26.63, 2.32** |

(continued)

| | Parameters at fa = 50% | | | | |
|---|---|---|---|---|---|
| Compounds | Dm | m | r | CI at fa = 50% | DRI# at fa = 50% |
| Cisplatin + DMC (1:16.57) | 8.6391 | 1.5212 | 0.9899 | 0.7007 | 50.03,1.47 |
| All CI values < 1, indicating synergistic effects between cisplatin & DMC<br>* The parameters m, Dm, and r are the slope, antilog of x-intercept, and the linear correlation coefficient of the median-effect plot, which signifies the shape of the dose-effect curve, the potency ($IC_{50}$), and conformity of the data to the mass-action law, respectively; 3 sets of dose-effect relationship experiments were carried out, each of which consists of 6-8 replicates.<br># DRI (dose reduction index) represents the order of magnitude (fold) of dose reduction that is allowed in combination for a given degree of effect as compared with the dose of each drug alone. All DRI values are calculated on the basis of the classic isobologram equation and assumptions. | | | | | |

**Example 2: Carboplatin in combination with DMC**

The combination of platinum anticancer drugs with demethylcantharidin or its hydrolyzed form (the diacid), in different proportions, show a synergistic effect in both sensitive and cisplatin-resistant tumorous cells. For example, as a result of synergy, the dose of carboplatin needed to achieve an antitumor effect (equivalent to carboplatin alone), may be reduced by twofold to eight fold when given in combination with demethylcantharidin in a sensitive L1210 cell line (Table 5). For comparable ratios of carboplatin and DMC, this dose reduction is substantially more dramatic in a cisplatin-resistant L1210 cell line (40 fold resistant to cisplatin). That is, the dose of carboplatin needed to achieve an equivalent antitumor effect is reduced by 17-fold (Carboplatin DMC at 1:1 by mole) to 65-fold (Carboplatin : DMC at 1:7 by mole) (Table 6).

From Tables 5 and 6, the dose reduction indices (DRI) for carboplatin in cisplatin-resistant L1210 were generally greater than that in cisplatin-sensitive L1210 cell lines. This can explain why combining DMC with carboplatin can potentially overcome cisplatin resistance by the synergism (Figs 3 and 4) between carboplatin and DMC.

Dose-effect relationships of carboplatin and demethylcantharidin (DMC) and their combinations on growth inhibition of cisplatin, carboplatin or oxaliplatin-sensitive and - resistant L1210 mouse leukaemia cell line during 72-hr exposure

**Table 5: Sensitive parental L1210 mouse leukaemia**

| | Parameters at fa = 50% | | | | |
|---|---|---|---|---|---|
| Compounds | Dm | m | r | CI at fa = 50% | DRI# at fa = 50% |
| Carboplatin | 10.6240 | 1.0537 | 0.9986 | | |
| Demethylcantharidin (DMC) | 13.5715 | 2.4384 | 0.9918 | | |
| Carboplatin + DMC (13.58:1) | 10.8235 | 1.3560 | 0.9821 | 1.0036 | 1.05, 18.28 |
| Carboplatin + DMC (6.79:1) | 10.1711 | 1.3748 | 0.9925 | 0.9307 | 1.20, 10.39 |
| Carboplatin + DMC (2.72:1) | 9.9109 | 0.9783 | 0.9919 | 0.8784 | 1.47, 5.09 |
| Carboplatin + DMC (1.51:1) | 9.8939 | 1.1008 | 0.9712 | 0.8507 | 1.78,3.44 |
| **Carboplatin + DMC (1:1.10)** | 10.4729 | 1.3456 | 0.9834 | 0.8736 | **2.13, 2.47** |
| Carboplatin + DMC (1:1.84) | 8.3669 | 1.1390 | 0.9381 | 0.6767 | 3.61,2.50 |
| Carboplatin + DMC (1:3.31) | 11.6969 | 2.0156 | 0.9840 | 0.9174 | 3.91, 1.51 |
| **Carboplatin + DMC (1:7.74)** | 11.5793 | 1.9523 | 0.9568 | 0.8803 | **8.02, 1.32** |
| All CI values ≤ 1, indicating synergistic effects between carboplatin & DMC | | | | | |

**Table 6: Cisplatin- resistant L1210 mouse leukaemia** (~ 40-fold resistant to cisplatin)

| | Parameters at fa = 50% | | | | |
|---|---|---|---|---|---|
| Compounds | Dm | m | r | CI at fa=50% | DRI# at fa = 50% |
| Carboplatin | 154.174 | 0.8792 | 0.9768 | | |
| Demethylcantharidin (DMC) | 14.5686 | 1.5182 | 0.9937 | | |
| Carboplatin + DMC (6.79:1) | 46.4854 | 0.5125 | 0.9467 | 0.7054 | 3.38,2.44 |
| Carboplatin + DMC (3.77:1) | 32.2953 | 0.4541 | 0.9093 | 0.6511 | 5.37, 2.15 |
| Carboplatin + DMC (2.26:1) | 21.6435 | 0.6689 | 0.9555 | 0.5653 | 9.13, 2.19 |
| Carboplatin + DMC (1.36:1) | 13.4400 | 1.0366 | 0.9616 | 0.4474 | 17.69,2.56 |
| **Carboplatin + DMC (1:1.33)** | 19.0064 | 1.4948 | 0.9912 | 0.8042 | **16.79, 1.34** |
| Carboplatin + DMC (1:1.92) | 19.0150 | 1.4325 | 0.9956 | 0.9063 | 21.07, 1.16 |
| **Carboplatin + DMC (1:6.59)** | 15.9520 | 1.4956 | 0.9843 | 0.9660 | **65.16, 1.05** |
| All CI values < 1, indicating synergistic effects between carboplatin & DMC<br>*The parameters m, Dm, and r are the slope, antilog of x-intercept, and the linear correlation coefficient of the median-effect plot, which signifies the shape of the dose-effect curve, the potency ($IC_{50}$), and conformity of the data to the mass-action law, respectively; 3 sets of dose-effect relationship experiments were carried out, each of which consists of 6-8 replicates.<br># DRI (dose reduction index) represents the order of magnitude (fold) of dose reduction that is allowed in combination for a given degree of effect as compared with the dose of each drug alone. All DRI values are calculated on the basis of the classic isobologram equation and assumptions. | | | | | |

**Example 3: Oxaliplatin in combination with DMC**

The combination of oxaliplatin with demethylcantharidin or its hydrolyzed form (the diacid), in different proportions, show a synergistic effect in both sensitive and cisplatin-resistant tumorous cells. For example, as a result of synergy, the dose of oxaliplatin needed to achieve an antitumor effect (equivalent to oxaliplatin alone), may be reduced by roughly twofold when given in combination with demethylcantharidin in a sensitive L1210 cell line (Table 7). The dose reduction is greater in a cisplatin-resistant L1210 cell line (40 fold resistant to cisplatin), that is, the dose of oxaliplatin needed to achieve an equivalent antitumor effect can be reduced by fourfold (Table 8).

Dose-effect relationships of oxaliplatin and demethylcantharidin (DMC) and their combinations on growth inhibition of cisplatin, carboplatin or oxaliplatin-sensitive and - resistant L1210 mouse leukaemia cell line during 72-hr exposure

**Table 7: Sensitive parental L1210 mouse leukaemia**

| | Parameters at fa = 50% | | | | |
|---|---|---|---|---|---|
| Compounds | Dm | m | r | CI at fa = 50% | DRI# at fa = 50% |
| Oxaliplatin | 0.2368 | 0.8324 | 0.9395 | | |
| Demethylcantharidin (DMC) | 11.0759 | 2.2353 | 0.9993 | | |
| Oxaliplatin + DMC (5.08:1) | 0.2522 | 0.7817 | 0.9456 | 0.8935 | 1.12,266.9 |
| Oxaliplatin + DMC (2.54:1) | 0.2528 | 0.8140 | 0.9623 | 0.7725 | 1.31, 155.1 |
| Oxaliplatin + DMC (1.35:1) | 0.2975 | 0.7275 | 0.9793 | 0.7340 | 1.38,87.63 |
| **Oxaliplatin + DMC (1:1.18)** | 0.2970 | 0.6528 | 0.9950 | 0.5893 | **1.74, 68.84** |
| Oxaliplatin + DMC (1:2.22) | 0.6874 | 0.8513 | 0.9812 | 0.9456 | 1.11,23.39 |
| Oxaliplatin + DMC (1:3.94) | 1.0055 | 0.8835 | 0.9864 | 0.9322 | 1.16, 13.81 |

(continued)

| | Parameters at fa = 50% | | | | |
|---|---|---|---|---|---|
| Compounds | Dm | m | r | CI at fa = 50% | DRI# at fa = 50% |
| Oxaliplatin + DMC (1:7.38) | 1.2449 | 1.0597 | 0.9971 | 0.7261 | 1.59, 10.10 |
| All CI values < 1, indicating synergistic effects between oxaliplatin & DMC | | | | | |

**Table 8: Cisplatin-resistant L1210 mouse leukaemia** (~ 40-fold resistant to cisplatin)

| | Parameters at fa = 50% | | | | |
|---|---|---|---|---|---|
| Compounds | Dm | m | r | CI at fa = 50% | DRI# at fa = 50% |
| Oxaliplatin | 0.8062 | 0.6145 | 0.9689 | | |
| Demethylcantharidin (DMC) | 14.0306 | 1.1715 | 0.9864 | | |
| Oxaliplatin + DMC (5.08:1) | 0.9505 | 0.5760 | 0.9799 | 0.9961 | 1.02,89.71 |
| Oxaliplatin + DMC (2.54:1) | 0.8463 | 0.6193 | 0.9564 | 0.7701 | 1.33,58.68 |
| Oxaliplatin + DMC (1.35:1) | 0.7842 | 0.6021 | 0.9669 | 0.5833 | 1.79,42.12 |
| **Oxaliplatin + DMC (1:1.18)** | 0.4839 | 0.6134 | 0.9141 | 0.2938 | **3.63, 53.53** |
| Oxaliplatin + DMC (1:2.22) | 0.9703 | 0.5639 | 0.9991 | 0.4177 | 2.67, 20.99 |
| Oxaliplatin + DMC (1:3.94) | 1.5049 | 0.9877 | 0.9877 | 0.4636 | 2.65, 11.69 |
| Oxaliplatin + DMC (1:7.38) | 3.7125 | 0.8608 | 0.9993 | 0.7823 | 1.82,4.29 |
| All CI values < 1, indicating synergistic effects between oxaliplatin & DMC<br>* The parameters m, Dm, and r are the slope, antilog of x-intercept, and the linear correlation coefficient of the median-effect plot, which signifies the shape of the dose-effect curve, the potency ($IC_{50}$), and conformity of the data to the mass-action law, respectively; 3 sets of dose-effect relationship experiments were carried out, each of which consists of 6-8 replicates.<br># DRI (dose reduction index) represents the order of magnitude (fold) of dose reduction that is allowed in combination for a given degree of effect as compared with the dose of each drug alone. All DRI values are calculated on the basis of the classic isobologram equation and assumptions. | | | | | |

**Example 4: DMC-Pt Complex 1 in combination with DMC**

The combination of Complex 1 with demethylcantharidin or its hydrolyzed form (the diacid), in different proportions, show a synergistic effect in both sensitive and cisplatin-resistant tumorous cells. For example, as a result of synergy, the dose of complex 1 needed to achieve an antitumor effect (equivalent to Complex 1 alone), may be reduced by threefold to fivefold when given in combination with demethylcantharidin in a sensitive L1210 cell line (Table 9). For comparable ratios of Complex 1 and DMC, this dose reduction is substantially more dramatic in a cisplatin-resistant L1210 cell line (40 fold resistant to cisplatin). That is, the dose of Complex 1 needed to achieve an equivalent antitumor effect is reduced by 4-fold (Complex 1 : DMC at 1:1 by mole) to 17-fold (Complex 1: DMC at 1:5 by mole) (Table 10).

From Tables 9 and 10, the dose reduction indices (DRI) for Complex 1 in cisplatin-resistant L1210 were generally greater than that in cisplatin-sensitive L1210 cell lines. This can explain why combining DMC with Complex 1 can potentially overcome cisplatin resistance by the synergism (Figs 7 and 8) between Complex 1 and DMC.

Dose-effect relationships of Cpx 1 and demethylcantharidin (DMC) and their combinations on growth inhibition of sensitive and cisplatin-resistant L1210 mouse leukaemia cell line during 72-hr exposure*

**Table 9: Sensitive parental L1210 mouse leukaemia**

| | Parameters at fa = 50% | | | | |
|---|---|---|---|---|---|
| Compounds | Dm | m | r | CI at fa = 50% | DRI# at fa = 50% |
| Cpx 1 | 5.1469 | 1.7975 | 0.9777 | | |
| Demethylcantharidin (DMC) | 11.0324 | 2.6927 | 1.0000 | | |

(continued)

| | Parameters at fa = 50% | | | | |
|---|---|---|---|---|---|
| Compounds | Dm | m | r | CI at fa = 50% | DRI# at fa = 50% |
| Cpx 1 + DMC (12.2:1) | 5.4079 | 1.3747 | 0.9970 | 1.0082 | 1.03,26.93 |
| Cpx 1 + DMC (4.88:1) | 5.4820 | 1.5981 | 0.9975 | 0.9685 | 1.13, 11.83 |
| Cpx 1 + DMC (2.71:1) | 5.4585 | 1.2726 | 0.9380 | 0.9080 | 1.29, 7.50 |
| Cpx 1 + DMC (1.63:1) | 2.6370 | 1.4191 | 0.9349 | 0.4086 | 3.15, 10.98 |
| **Cpx 1 + DMC (1.02:1)** | 3.5632 | 1.0280 | 0.9844 | 0.5057 | **2.92, 6.11** |
| Cpx 1 + DMC (1:1.84) | 4.7534 | 1.2080 | 0.9945 | 0.6046 | 3.08,3.57 |
| **Cpx 1 + DMC (1:5.22)** | 6.255 | 1.3650 | 0.9946 | 0.6711 | **5.12,2.10** |
| All CI values < 1, indicating synergistic effects between Cpx 1 & DMC | | | | | |

**Table 10: Cisplatin-resistant L1210 mouse leukaemia** (~ 40-fold resistant to cisplatin)

| | Parameters at fa = 50% | | | | |
|---|---|---|---|---|---|
| Compounds | Dm | m | r | CI at fa=50% | DRI# at fa = 50% |
| Cpx 1 | 42.3683 | 2.7684 | 1.0000 | | |
| Demethylcantharidin (DMC) | 17.5153 | 2.5861 | 0.9888 | | |
| Cpx 1 + DMC (73.23:1) | 39.7635 | 1.9791 | 0.9995 | 0.9565 | 1.08,32.70 |
| Cpx 1 + DMC (36.61:1) | 39.3813 | 2.8198 | 1.0000 | 0.9646 | 1.11, 16.73 |
| Cpx 1 + DMC (14.64:1) | 27.4652 | 2.5140 | 0.9798 | 0.7070 | 1.65,9.98 |
| Cpx 1 + DMC (8.14:1) | 21.719 | 1.2451 | 0.9700 | 0.5922 | 2.19, 7.37 |
| Cpx 1 + DMC (4.88:1) | 21.0396 | 1.2983 | 0.9471 | 0.6164 | 2.43,4.90 |
| Cpx 1 + DMC (2.93:1) | 18.5376 | 1.2117 | 0.9203 | 0.5955 | 3.07,3.71 |
| **Cpx 1 + DMC (1.63:1)** | 16.1768 | 1.2377 | 0.9917 | 0.5878 | **4.23, 2.85** |
| **Cpx 1 + DMC (1:5.42)** | 16.0850 | 1.2235 | 0.9935 | 0.8345 | **16.91,1.29** |
| All CI values < 1, indicating synergistic effects between Cpx 1 & DMC<br>*The parameters m, Dm, and r are the slope, antilog of x-intercept, and the linear correlation coefficient of the median-effect plot, which signifies the shape of the dose-effect curve, the potency ($IC_{50}$), and conformity of the data to the mass-action law, respectively; n is the number of sets of dose-effect relationship experiments that were carried out.<br># DRI (dose reduction index) represents the order of magnitude (fold) of dose reduction that is allowed in combination for a given degree of effect as compared with the dose of each drug alone. All DRI values are calculated on the basis of the classic isobologram equation and assumptions. | | | | | |

**Example 5: DMC-Pt Complex 5 in combination with DMC**

The combination of Complex 5 with demethylcantharidin or its hydrolyzed form (the diacid), in different proportions, show only a mild synergistic effect in both sensitive and cisplatin-resistant tumorous cells (Figs 9 and 10).

From Tables 11 and 12, the dose reduction indices (DRI) for Complex 1 in cisplatin-resistant L1210 were roughly the same as that in cisplatin-sensitive L1210 cell lines. It is observed that even at high proportions of DMC, the DRI for Complex 5 remains small, unlike the results observed for cisplatin (Example 1) and carboplatin (Example 2). This implies that Complex 5 alone can overcome cisplatin-induced resistance in tumorous cells and that Complex 5 alone is a highly effective anticancer agent.

Dose-effect relationships of Cpx 5 and demethylcantharidin (DMC) and their combinations on growth inhibition of sensitive and cisplatin-resistant L1210 mouse leukaemia cell line during 72-hr exposure*

**Table 11: Sensitive parental L1210 mouse leukaemia**

| | Parameters at fa = 50% | | | | |
|---|---|---|---|---|---|
| Compounds | Dm | m | r | CI at fa = 50% | DRI# at fa = 50% |
| Cpx 5 | 0.2542 | 0.9498 | 0.9818 | | |
| Demethylcantharidin (DMC) | 15.4781 | 1.4432 | 0.9278 | | |
| **Cpx 5 + DMC (1.02:1)** | 0.3455 | 0.8636 | 0.9822 | 0.6975 | **1.46, 90.52** |
| Cpx 5 + DMC (1:1.96) | 0.6184 | 0.8968 | 0.9981 | 0.8483 | 1.22,37.80 |
| **Cpx 5 + DMC (1:4.89)** | 0.8633 | 1.0540 | 0.9930 | 0.6230 | **1.73,21.60** |
| Cpx 5 + DMC (1:8.80) | 1.0180 | 0.7496 | 0.9904 | 0.4678 | 2.45, 16.93 |
| Cpx 5 + DMC (1:14.67) | 1.5976 | 1.1217 | 0.9850 | 0.4975 | 2.49, 10.35 |
| Cpx 5 + DMC (1:24.46) | 3.3904 | 1.6365 | 0.9891 | 0.7344 | 1.91, 4.74 |
| Cpx 5 + DMC (1:44.03) | 3.4040 | 1.6365 | 0.9946 | 0.5124 | 3.36, 4.65 |
| All CI values < 1, indicating synergistic effects between cpx 5 & DMC | | | | | |

**Table 12: Cisplatin-resistant L1210 mouse leukaemia** (~ 40-fold resistant to cisplatin)

| | Parameters at fa = 50% | | | | |
|---|---|---|---|---|---|
| Compounds | Dm | m | r | CI at fa = 50% | DRI# at fa = 50% |
| Cpx 5 | 0.3983 | 0.7887 | 0.9326 | | |
| Demethylcantharidin (DMC) | 16.6914 | 1.6832 | 0.9889 | | |
| **Cpx 5 + DMC (1.02:1)** | 0.3887 | 0.6272 | 0.9839 | 0.5044 | **2.03, 86.75** |
| Cpx 5 + DMC (1:1.96) | 0.6909 | 0.6633 | 0.9975 | 0.6134 | 1.71,36.48 |
| Cpx 5 + DMC (1:4.89) | 1.4376 | 1.0899 | 0.9974 | 0.6844 | 1.63, 13.99 |
| **Cpx 5 + DMC (1:8.80)** | 2.6459 | 1.2940 | 0.9968 | 0.8199 | **1.48, 7.03** |
| Cpx 5 + DMC (1:14.67) | 3.9345 | 1.5512 | 0.9983 | 0.8511 | 1.59, 4.53 |
| Cpx 5 + DMC (1:24.46) | 4.2268 | 1.3019 | 0.9954 | 0.6601 | 2.41, 4.11 |
| Cpx 5+DMC (1:44.03) | 5.9462 | 1.8555 | 0.9917 | 0.6797 | 3.02, 2.87 |
| All CI values < 1, indicating synergistic effects between cpx 5 & DMC<br>* The parameters m, Dm, and r are the slope, antilog of x-intercept, and the linear correlation coefficient of the median-effect plot, which signifies the shape of the dose-effect curve, the potency ($IC_{50}$), and conformity of the data to the mass-action law, respectively; n is the number of sets of dose-effect relationship experiments that were carried out.<br># DRI (dose reduction index) represents the order of magnitude (fold) of dose reduction that is allowed in combination for a given degree of effect as compared with the dose of each drug alone. All DRI values are calculated on the basis of the classic isobologram equation and assumptions. | | | | | |

**Summary of Cross-Resistance of Pt-containing anticancer agents to Cisplatin**

**Table 13: Dose-effect relationships of Pt-containing anticancer agents and demethylcantharidin (DMC) on growth inhibition of sensitive and cisplatin-resistant L1210 mouse leukaemia cell line during 72-hr exposure* #**

| Compounds | Dm (i.e. $IC_{50}$)<br>Sensitive cell L1210<br>CDDP-resistant L1210<br>(fold-resistant) | m | r |
|---|---|---|---|
| **Cisplatin** | 0.6031<br>24.6021 (40.8) | 1.2405<br>1.6239 | 0.9884<br>0.9958 |
| **Carboplatin** | 10.6240<br>154.174 (14.5) | 1.0537<br>0.8972 | 0.9986<br>0.9768 |
| **Complex 1** | 5.1469<br>42.3683 (8.2) | 1.7975<br>2.7684 | 0.9777<br>1.0000 |
| **Complex 5** | 0.2542<br>0.3983 (1.6) | 0.9498<br>0.7887 | 0.9818<br>0.9326 |
| **Oxaliplatin** | 0.2368<br>0.8062 (3.4) | 0.8324<br>0.6145 | 0.9395<br>0.9689 |
| **DMC** | 12.8277<br>14.3149 (1.1) | 1.8366<br>1.6107 | 0.9807<br>0.9852 |
| * The parameters m, Dm, and r are the slope, antilog of x-intercept, and the linear correlation coefficient of the median-effect plot, which signifies the shape of the dose-effect curve, the potency ($IC_{50}$), and conformity of the data to the mass-action law, respectively; 3 sets of dose-effect relationship experiments were carried out, each of which consists of 6-8 replicates.<br># The parameters were evaluated at fa = 50% using the median effect plot. | | | |

The acquired resistant cell line is ~ 40-fold resistant to cisplatin & ~ 15-fold cross-resistant to carboplatin. Oxaliplatin & complex 1 also show different degree of cross-resistant. However, complex 5 & demethylcantharidin (DMC) show negligible cross-resistance.

**Summary of Dose-reduction index (DRI) for Pt-containing anticancer agents**

**Table 14: Dose-effect relationships of combinations (in about 1:1 molar ratio) of various platinum compounds & demethylcantharidin (DMC) on growth inhibition of sensitive and cisplatin-resistant L1210 mouse leukaemia cell line during 72-hr exposure*#**

| | Parameters at fa = 50%<br>Sensitive cell line (1st row) Resistant cell line (2nd row) | | | | |
|---|---|---|---|---|---|
| **Combinations** | **Dm** | **m** | **r** | **CI at fa = 50%** | **DRI# at fa = 50%** |
| **Cisplatin + DMC**<br>**(sensitive -1: 1.49)**<br>**(resistant -1: 1.67)** | 1.3018<br>10.243 | 1.3556<br>1.7571 | 0.9849<br>0.9927 | 0.9365<br>0.6915 | 1.15, 14.36<br>6.42, 1.87 |
| **Carboplatin + DMC** | | | | | |

(continued)

| Combinations | Dm | m | r | CI at fa = 50% | DRI# at fa = 50% |
|---|---|---|---|---|---|
| **(sensitive - 1.51:1)** | 9.8939 | 1.1008 | 0.9712 | 0.8507 | 1.78, 3.44 |
| **(resistant -1.36:1)** | 13.4400 | 1.0366 | 0.9616 | 0.4474 | 17.69, 2.56 |
| **Complex 1 + DMC** | | | | | |
| **(sensitive -1.63:1)** | 2.6370 | 1.4191 | 0.9349 | 0.4086 | 3.15, 10.98 |
| **(resistant -1.63:1)** | 16.1768 | 1.2377 | 0.9942 | 0.5878 | 4.23, 2.85 |
| **Complex 5 + DMC** | | | | | |
| **(sensitive - 1.02:1)** | 0.3455 | 0.8636 | 0.9822 | 0.6975 | 1.46, 90.52 |
| **(resistant - 1.02:1)** | 0.3887 | 0.6272 | 0.9839 | 0.9839 | 2.03, 86.75 |
| **Oxaliplatin + DMC** | | | | | |
| **(sensitive - -1: 1.18)** | 0.2970 | 0.6528 | 0.9950 | 0.5893 | 1.74, 68.84 |
| **(resistant -1: 1.18)** | 0.4839 | 0.6134 | 0.9141 | 0.2938 | 3.63, 53.53 |
| * The parameters m, Dm, and r are the slope, antilog of x-intercept, and the linear correlation coefficient of the median-effect plot, which signifies the shape of the dose-effect curve, the potency ($IC_{50}$), and conformity of the data to the mass-action law, respectively; 3 sets of dose-effect relationship experiments were carried out, each of which consists of 6-8 replicates.<br># The parameters were evaluated at fa = 50% using the median effect plot. | | | | | |

For compounds showing cross-resistance to cisplatin [cisplatin (40-fold), carboplatin (15-fold), Complex 1 (8-fold)], the CI in the cisplatin-resistant cell line is generally lower than that in the parental cell line whereas DRI in the cisplatin-resistant cell line is generally greater than that in the parental cell line. These data suggests that DMC is targeting preferentially towards the resistance mechanism.

## Experimental Details

### Preparation of hydrolyzed DMC:

1 part of DMC was allowed to dissolve in 2 parts of 1M sodium hydroxide. The resulting solution was dried *in vacuo.* The white solid formed was characterized by FT-IR and NMR.

### In vitro antitumor-drug screening

Test complexes 1-5, DMC, hydrolyzed DMC, cantharidin, together with controls cisplatin and carboplatin, were

screened for *in vitro* antitumor activity against a series of human cancer cell lines and mouse leukaemia L1210, using a tetrazolium microculture assay.

**Drugs**: Drugs to be tested were dissolved in complete medium at twice the highest concentration to be used, followed by filter (0.22μm) sterilization. A series of log dilutions were made of each drug solution using complete medium. All drug solutions were prepared within 10 minutes before adding to the cells.

**Cells**: MDA-MB-231 (human breast), SK-OV-3 (human ovarian), SK-Hep-1 (human liver), COL0320DM (human colon), L1210 (mouse leukaemia), NCI:H460 (human lung) and NTERA-Scl-D (human testicular) were obtained from the American Type Culture Collection (ATCC).

**In vitro drug exposure**: Trypsinized cells were adjusted to a concentration of 2 x $10^5$ cells/ml in complete medium and for each drug to be tested, 0.1ml of the cell suspension was added to each of the 24 wells of a 96-well microtitre plate. The plate was then incubated at standard conditions for 24 hours in order to allow the cells to attach to the bottom of the well and resume exponential growth. To each well of the control cultures and drug/concentration combination, 0.1ml of complete medium and drug solution respectively was added. Each drug/concentration combination consisted of 4 replicates while control cultures consisted of 12 replicates. 12 replicates of blank consisting of 0.2ml complete medium were set aside to account for the background. The cultures were incubated for an additional 72 hours. All experiments were repeated at least 3 times on different occasions.

**MTT assay**: The MTT (tetrazolium) assay was used to determine the growth-inhibitory effects of different drugs. A 5 mg/ml solution of MTT (3-(4,5-dimethylthiazol-2-yl)-2,5-diphenyltetrazolium bromide) was prepared in phosphate buffer saline (pH 7.4), filter (0.22μm) sterilized and stored at -20°C. To each well, 0.02ml of the MTT solution was added and the plates were incubated at standard conditions for 4 hours. The media from each well was then removed and 0.1ml of DMSO (dimethylsulfoxide) was added to each well to solubilize the colored dye produced from the metabolic reduction of MTT. The amount of reduced MTT was determined by spectrophotometric means (570nm).

**Data analysis**: The mean OD for all 12 control wells was determined. For each concentration of each drug, the mean OD of the 4 wells was determined. All mean OD was subtracted from the blank to account for the background. The "Percent of Control" values were determined and plotted. The concentration of drug resulting in 50% growth inhibition (IC50) was determined using the software Prism 3.0 by fitting the graph into sigmoidal dose-response curve.

**Induction of cisplatin-resistance in L1210 mouse leukaemia**

The cisplatin-resistant L1210 leukaemia cell line was developed by exposing the L1210/0 cells to stepwise increments in drug concentration for over a year (Table 14).

**Synergism of combinations of platinum anticancer drugs with demethyleantharidin (DMC) or its hydrolyzed form, the diacid**

Platinum compounds were evaluated alone and in combination with demethylcantharidin (DMC) for their cytotoxic effects. The cytotoxicity for the compounds was determined by the MTT assay as described above. The median-effect principle and combination-index isobologram method of Chou and Talalay was used to determine the dose-effect parameters for cisplatin, DMC, or their mixtures at different combination ratios. Each drug or mixture was 3-fold serially diluted to generate a dose-effect relationship in the cytotoxic assays.

*Details for the assessment of synergy are as follows:*

Combinations of cisplatin and DMC were evaluated for synergism, additive effect or antagonism using the ***median-effect plot and combination-index isobologram method*** of Chou and Talalay references 1-4.

This method involves plotting dose-effect curves for each agent and their combinations in multiply-diluted concentrations by using the median-effect equation [3,6]

$$fa/fu = (D/Dm)^m$$

where **fa** is the fraction of cells affected (ie, fraction of cells killed), **fu** the fraction unaffected (i.e. fraction of cells surviving; fa = 1-fu), D the dose of drug and Dm the dose causing the median effect

The parameters m, Dm, and r are the slope, antilog of x-intercept, and the linear correlation coefficient of the median-effect plot, which signifies the shape of the dose-effect curve, the potency ($IC_{50}$), and conformity of the data to the mass-action law, in references 3, 5 and 6, respectively. Dm and m values are used for calculating the combination index (CI) values.

CI<1, CI=1, and CI>1 indicate synergism, additivity, and antagonism, respectively. As based on the classic isobologram equation, CI can be calculated by equation:

$$CI = [(D)_1/(D_x)_1] + [(D)_2/(D_x)_2] + [\alpha(D)_1(D)_2]/[(D_x)_1(D_x)_2],$$

where (D), is the dose of drug 1,
$(D)_2$ is the dose of drug 2 and
$(D_x)$ is the dose required to produce a median effect (= Dm$[fa/(1-fa)]^{1/m}$) $\alpha = 0$ for drugs with mutually exclusive and $\alpha = 1$ for drugs with mutually non-exclusive mechanisms of action

Both the mutually non-exclusive and exclusive drug interaction as defined by Chou and Chou (reference 7) were evaluated. As there were no substantial differences between the data for the two assumptions, the data provided are given for the mutually exclusive assumption only.

To preclude a false-positive result, ***classical isobologram curve tracing*** was used to confirm the synergistic effect at a reasonable fractional cytotoxic effect (fa = 50%).

**References cited above:**

1. Chou TC, Talalay P. A simple generalized equation for the analysis of multiple inhibitions of Michaelis-Menten kinetics systems. JBiol Chem 1977, 252, 6438-42.
2. Chou TC, Talalay P. Generalized equations for the analysis of inhibitions of Michaelis-Menten and higher-order kinetics systems with two or more mutually exclusive and nonexclusive inhibitors. Eur J Biochem 1981, 115, 207-16.
3. Chou TC, Talalay P. Quantitative analysis of dose-effect relationships: the combined effects of multiple drugs or enzyme inhibitors. Adv Enzyme Regul 1984, 22: 27-55.
4. Chou TC, Talalay P. Applications of the median-effect principle for the assessment of low-dose risk of carcinogens and for the quantitation of synergism and antagonism of chemotherapeutic agents. In New Avenues in Development Cancer Chemotherapy, Bristol-Myers Symposium series (Harrap KR, Connors TA, eds). New York: Academic Press, 1987, pp 37-64.
5. Chou TC. Derivation and properties of Michaelis-Menton type and Hill type equations for reference ligands. J Theor Biol 1976, 59, 253-76.
6. Chou TC. The median-effect principle and the combination index for quantitation of synergism and antagonism, chap 2. In Synergism and Antagonism in Chemotherapy (Chou TC, Rideout DC, eds) New York: Academic Press, 1991, pp 61-102.
7. Chou J, Chou TC. Dose-effect analysis with microcomputers: quantitation of ED50, LD50, synergism, antagonism, low dose risk, receptor-ligand and enzyme kinetics. EurJBiochem. Manual and Software, Biosoft, Cambridge, UK, 1987.


**Dose-Reduction Index**

The term "dose-reduction index" or DRI was first introduced in 1988 (Chou and Chou, 1988) and was used in 1989 for computerized automated analysis (Berman et al., 1989). DRI describes how many folds of dose reduction are allowed in a synergistic combination at a given degree effect (i.e. % inhibition) and at a given combination ratio.

$$CI = (D)_1/(D_x)_1 + (D)_2/(D_x)_2 = \mathbf{1/(DRI)_1 + 1/(DRI)_2}$$

where (D), is the dose of drug 1 in the combination,
$(D)_2$ is the dose of drug 2 in the combination and
$(D_x)$ is the dose (for the drug alone)

required to produce a median effect (= Dm$[fa/(1-fa)]^{1/m}$)

Therefore, for example,
in sensitive L1210 Cisplatin:DMC (1:1.49)

$$CI = \underline{0.523/0.603} + \underline{0.779/11.185} = 0.936$$

$$\downarrow \qquad\qquad \downarrow$$

$$1/DRI_{cisplatin} \qquad 1/DRI_{DMC}$$

$$\textbf{i.e. } DRI_{cisplatin} = 1.15\ ;\ DRI_{DMC} = 14.36$$

in cisplatin-resistant L1210 Cisplatin:DMC(1:1.67)

$$CI = \underline{3.836/24.602} + \underline{6.407/11.969} = 0.6915$$

$$\downarrow \qquad\qquad \downarrow$$

$$1/DRI_{cisplatin} \qquad 1/DRI_{DMC}$$

$$\textbf{i.e. } DRI_{cisplatin} = 6.42;\ DRI_{DMC} = 1.87$$

The DRI for cisplatin is about 5.5-fold higher in the resistant cell line than in the sensitive parental cell line. This may explain why combining DMC with cisplatin can potentially overcome cisplatin resistance.

References cited above:


- Chou J., Chou T.-C. Computerized simulation of dose reduction index (DRI) in synergistic drug combinations. Pharmacologist 1988; 30: Abstract A231.
- Berman E., Duigou-Osterndorf R., Krown S.E., Fanucchi M.P., Chou J., Hirsch M.S., Clarkson B.D., Chou T.-C. Synergistic cytotoxic effect of azidothymidine and recombinant interferon alpha on normal human bone marrow progenitor cells. Blood 1989; 74: 1281-6.


## Fa-CI plot

A fa-CI plot (Figure 11) can also be constructed to indicate synergism or antagonism of the two drugs at various effect levels in a mixture that is serially diluted (i.e. along the whole inhibition curve). When several mixtures are made, it is possible to estimate the optimal combination ratio for maximal synergy from the plot. Different effect levels usually give different degrees of synergism or antagonism. CI<1, =1, >1 indicate synergism, additive effect, and antagonism, respectively. For anticancer chemotherapy, synergism at high effect levels is clinically more relevant than synergism at low effect levels.

## Claims

1. Pharmaceutical composition, comprising a combination of demethylcantharidin (DMC) or its hydrolyzed form with a platinum anticancer agent having a synergistic effect in inhibiting the growth of cisplatin, carboplatin or oxaliplatin-sensitive and -resistant tumorous cells and a pharmaceutically acceptable carrier, wherein a molar ratio between demethylcantharidin or its hydrolysed form and the platinum anticancer agent is from 55:1 to 1:100.

2. The pharmaceutical composition according to claim 1, wherein the tumour cells are derived from lung, liver, breast, colon, ovary, cervix, leukaemia, lymphoma, or naso-pharyngeal carcinoma.

3. The pharmaceutical composition according to claim 1 or 2, wherein said platinum anticancer agent is selected from at least one of the complexes having structures of:

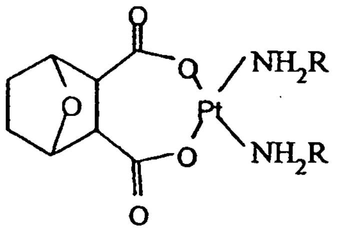

Structure I

wherein R=H,C1-C10 or ring size C3-C6;

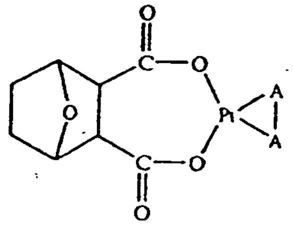

Structure II

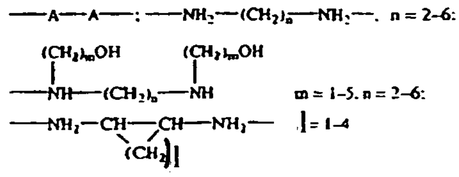

4. The pharmaceutical composition according to claim 3, wherein R is selected from H, $CH(CH_3)_2$, $CH_3$, and $C_2H_5$, and -A-A- is

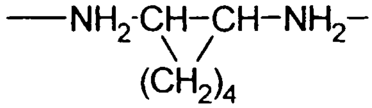

5. The pharmaceutical composition according to any one of claims 1 to 4, wherein said platinum anticancer agent is selected from cisplatin, carboplatin and oxaliplatin.

6. The pharmaceutical composition according to any of claims 1 to 5, wherein the molar ratio is from 10:1 1 to 1:10.

7. The pharmaceutical composition according to claim 6, wherein the molar ratio is 1:1.

8. Use of a combination of demethylcantharidin (DMC) or its hydrolysed form with a platinum anticancer agent for the production of a medicament for inhibiting of the growth of cisplatin, carboplatin or oxaliplatin-sensitive and -resistant tumorous cells in a subject, wherein a molar ratio between demethylcantharidin or its hydrolysed form and the platinum anticancer agent is from 55:1 to 1:100.

9. Use according to claim 9, wherein the tumour cells are derived from lung, liver, breast, colon, ovary, cervix, leukaemia, lymphoma, or naso-pharyngeal carcinoma.

10. Use according to claim 8 or 9, wherein the molar ratio is from 10:1 to 1:10, preferably 1:1.

**Patentansprüche**

1. Pharmazeutische Zusammensetzung, umfassend eine Kombination aus Demethylcantharidin (DMC) oder seine hydrolysierte Form mit einem Platin-Antikrebsmittel, wobei die Kombination einen synergistischen Effekt bei der Inhibierung des Wachstums von Cisplatin-, Carboplatin- oder Oxaliplatin-sensitiven und -resistenten Tumorzellen aufweist, und einen pharmazeutisch akzeptablen Träger, wobei ein molares Verhältnis zwischen Demethylcantharidin oder seiner hydrolysierten Form und dem Platin-Antikrebsmittel von 55:1 bis 1:100 vorliegt.

2. Pharmazeutische Zusammensetzung gemäß Anspruch 1, wobei die Tumorzellen von Lunge, Leber, Brust, Kolon, Eierstock, Zervix, Leukämie, Lymphoma oder Nasopharyngeal-Karzinom abgeleitet werden.

3. Pharmazeutische Zusammensetzung gemäß Anspruch 1 oder 2, wobei das Platin-Antikrebsmittel aus mindestens

einem der Komplexe ausgewählt ist, der die Struktur von:

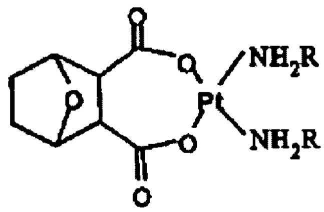

**Struktur I**

wobei R = H, C1 - C 10 oder Ringgröße C3 - C6,

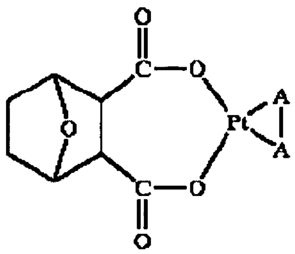

**Struktur II**

-A-A- : $-NH_2-(CH_2)_n-NH_2-$. n = 2-6;

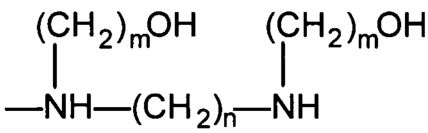

m = 1-5. n = 2-6;

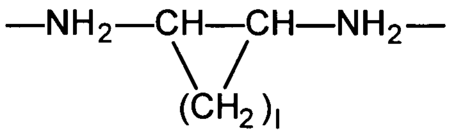

l = 1-4;

aufweist.

**4.** Pharmazeutische Zusammensetzung gemäß Anspruch 3, wobei R aus H, $CH(CH_3)_2$, $CH_3$, und $C_2H_5$ ausgewählt ist und -A-A-

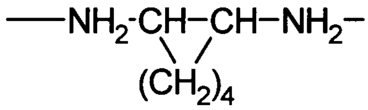

ist.

**5.** Pharmazeutische Zusammensetzung gemäß einem der Ansprüche 1 bis 4, wobei das Platin-Antikrebsmittel aus Cisplatin, Carboplatin und Oxaliplatin ausgewählt ist.

**6.** Pharmazeutische Zusammensetzung gemäß einem der Ansprüche 1 bis 5, wobei das molare Verhältnis von 10:1 1 bis 1:10 ist.

**7.** Pharmazeutische Zusammensetzung gemäß Anspruch 6, wobei das molare Verhältnis 1:1 ist.

**8.** Verwendung einer Kombination von Demethylcantharidin (DMC) oder seiner hydrolysierten Form mit einem Platin-Antikrebsmittel für die Herstellung eines Medikaments zur Inhibierung des Wachstums von Cisplatin-, Carboplatin- oder Oxaliplatin-sensitiven und -resistenten Tumorzellen in einem Subjekt, wobei ein molares Verhältnis zwischen Demethylcantharidin oder seiner hydrolysierten Form und dem Platin-Antikrebsmittel von 55:1 bis 1:100 vorliegt.

**9.** Verwendung gemäß Anspruch 9, wobei die Tumorzellen von Lunge, Leber, Brust, Kolon, Eierstock, Zervix, Leukämie, Lymphoma oder Nasopharyngeal-Karzinom abgeleitet werden.

**10.** Verwendung gemäß Anspruch 8 oder 9, wobei das molare Verhältnis von 10:1 bis 1:10, bevorzugt 1:1 ist.

## Revendications

**1.** Composition pharmaceutique comprenant une association de déméthylcantharidine (DMC) ou de sa forme hydrolysée avec un agent anticancéreux à base de platine ayant un effet synergique sur l'inhibition de la croissance de cellules tumorales sensibles ou résistantes au cisplatine, au carboplatine ou à l'oxaliplatine et d'un support pharmaceutiquement acceptable, dans laquelle un rapport molaire de la déméthylcantharidine ou de sa forme hydrolysée à l'agent anticancéreux à base de platine est de 55/1 à 1/100.

**2.** Composition pharmaceutique selon la revendication 1, dans laquelle les cellules tumorales sont dérivées d'un cancer des poumons, du foie, du sein, du colon, des ovaires, du col de l'utérus, d'une leucémie, d'un lymphome ou d'un cancer du rhinopharynx.

**3.** Composition pharmaceutique selon la revendication 1 ou 2, dans laquelle ledit agent anticancéreux à base de platine est choisi parmi au moins un des complexes ayant les structures :

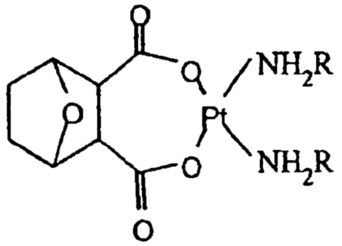

Structure I
où R=H,C1-C10 ou cycle en C3-C6

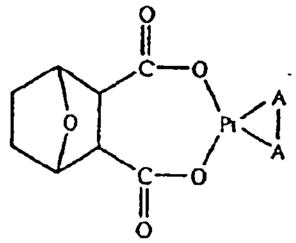

Structure II

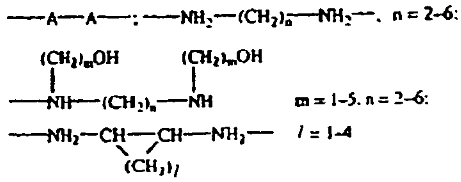


**4.** Composition pharmaceutique selon la revendication 3, dans laquelle R est choisi parmi H, $CH(CH_3)_2$, $CH_3$, et $C_2H_5$, et - A-A- est

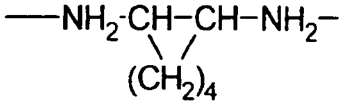

.

**5.** Composition pharmaceutique selon l'une quelconque des revendications 1 à 4, dans laquelle ledit agent anticancéreux à base de platine est choisi parmi le cisplatine, le carboplatine et l'oxaliplatine.

**6.** Composition pharmaceutique selon l'une quelconque des revendications 1 à 5, dans laquelle le rapport molaire est de 10/1 à 1/10.

**7.** Composition pharmaceutique selon la revendication 6,
dans laquelle le rapport molaire est de 1/1.

**8.** Utilisation d'une association de déméthylcantharidine (DMC) ou de sa forme hydrolysée avec un agent anticancéreux à base de platine pour la production d'un médicament destiné à inhiber la croissance de cellules tumorales sensibles ou résistantes au cisplatine, au carboplatine ou à l'oxaliplatine chez un sujet, dans laquelle un rapport molaire de la déméthylcantharidine ou de sa forme hydrolysée à l'agent anticancéreux à base de platine est de 55/1 à 1/100.

**9.** Utilisation selon la revendication 9, dans laquelle les cellules tumorales sont dérivées d'un cancer des poumons, du foie, du sein, du colon, des ovaires, du col de l'utérus, d'une leucémie, d'un lymphome ou d'un cancer du rhinopharynx.

**10.** Utilisation selon la revendication 8 ou 9, dans laquelle le rapport molaire est de 10/1 à 1/10, de préférence de 1/1.

**Fig. 1**

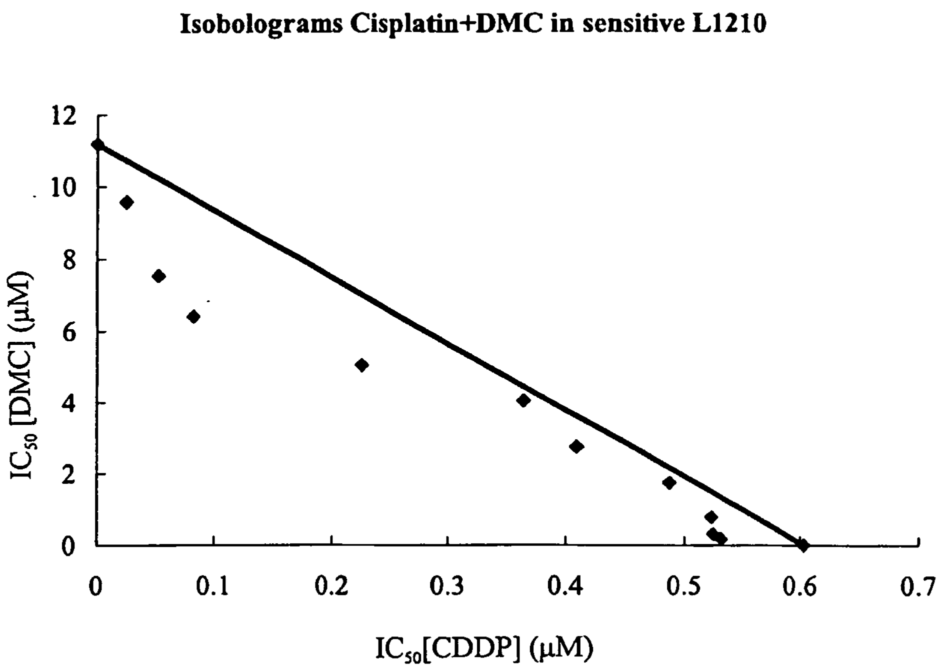
Isobolograms Cisplatin+DMC in sensitive L1210
12
10
8
6
4
2
0
0
0.1
0.2
0.3
0.4
0.5
0.6
0.7
$IC_{50}$ [DMC] (μM)
$IC_{50}$[CDDP] (μM)

**Fig. 2**

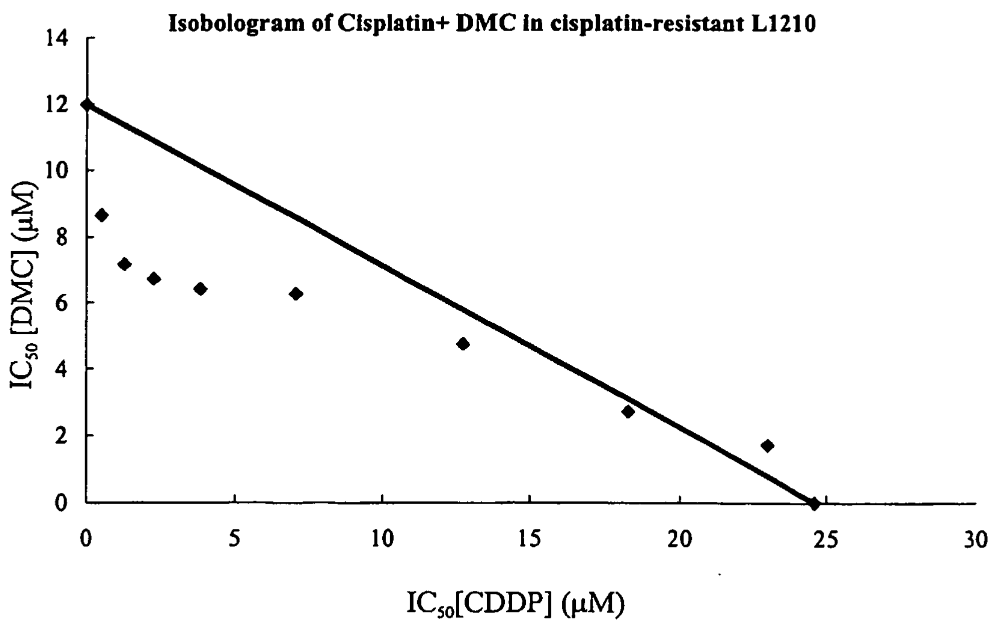
Isobologram of Cisplatin+ DMC in cisplatin-resistant L1210
14
12
10
8
6
4
2
0
0
5
10
15
20
25
30
$IC_{50}$ [DMC] (μM)
$IC_{50}$[CDDP] (μM)

Fig. 3

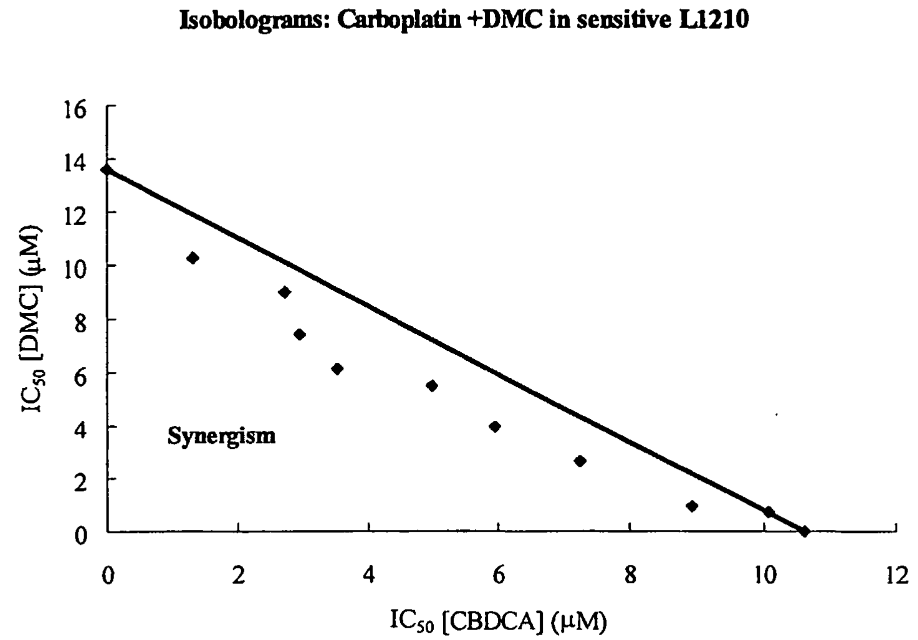
Isobolograms: Carboplatin +DMC in sensitive L1210
IC50 [DMC] (μM)
Synergism
IC50 [CBDCA] (μM)
0
2
4
6
8
10
12
14
16
0
2
4
6
8
10
12

Fig. 4

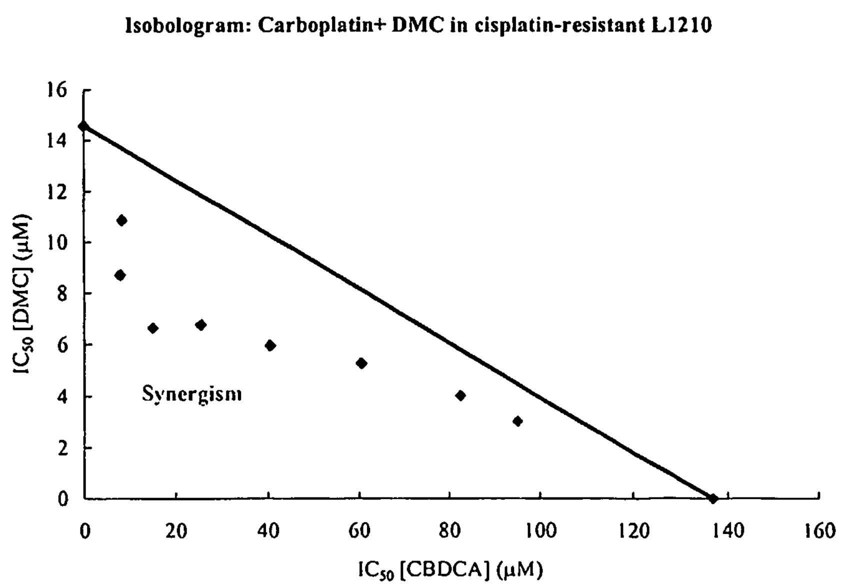
Isobologram: Carboplatin+ DMC in cisplatin-resistant L1210
IC50 [DMC] (μM)
Synergism
IC50 [CBDCA] (μM)
0
2
4
6
8
10
12
14
16
0
20
40
60
80
100
120
140
160

Fig. 5

**Isobolograms: Oxaliplatin +DMC in sensitive L1210**

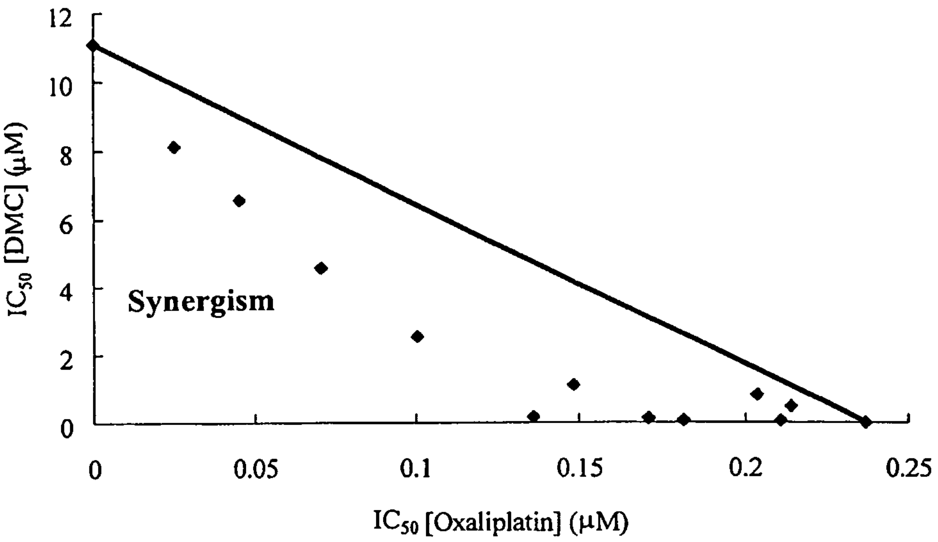


Fig. 6

**Isobologram: Oxaliplatin+ DMC in cisplatin-resistant L1210**

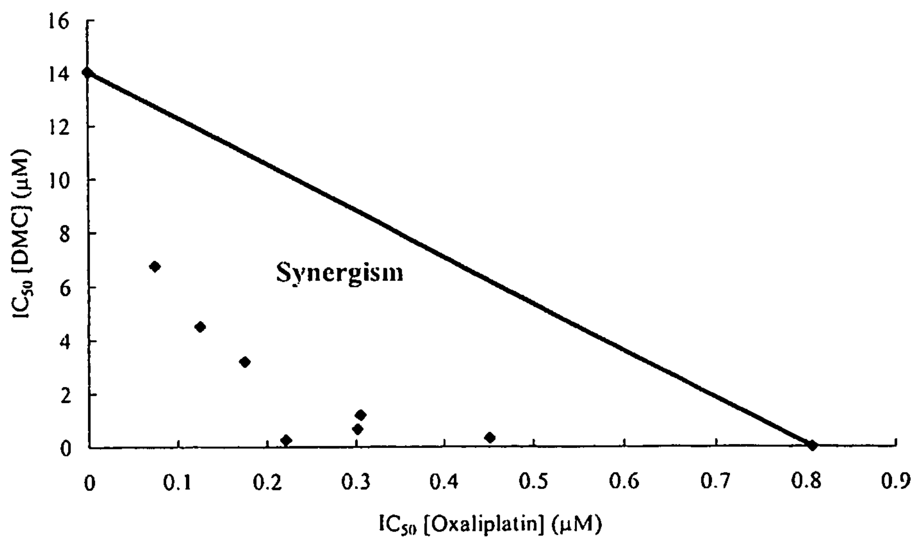

Fig. 7

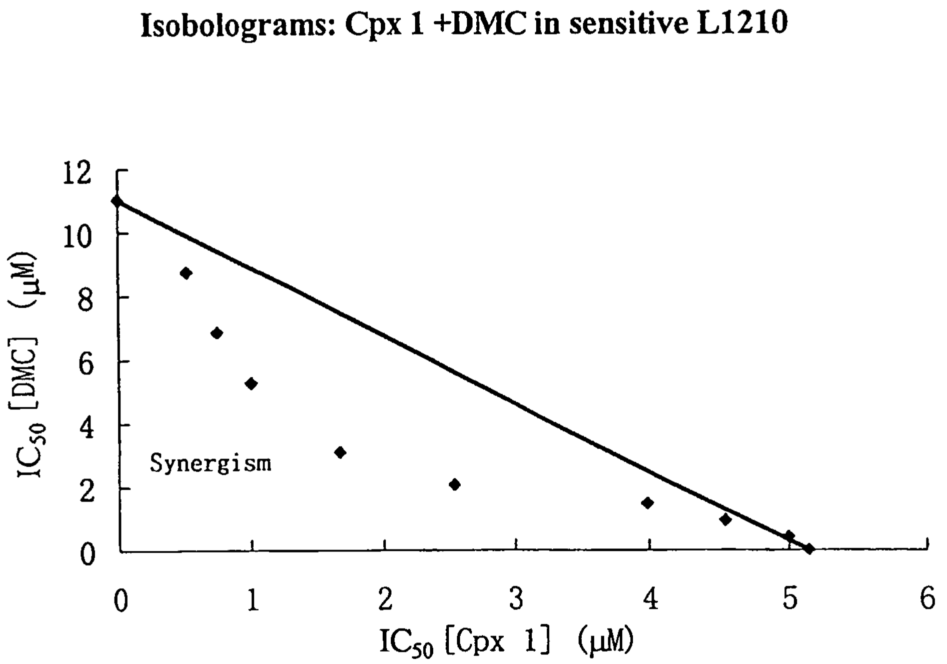
Isobolograms: Cpx 1 +DMC in sensitive L1210
12
10
8
6
4
2
0
IC50 [DMC] (μM)
Synergism
0
1
2
3
4
5
6
IC50 [Cpx 1] (μM)

Fig. 8

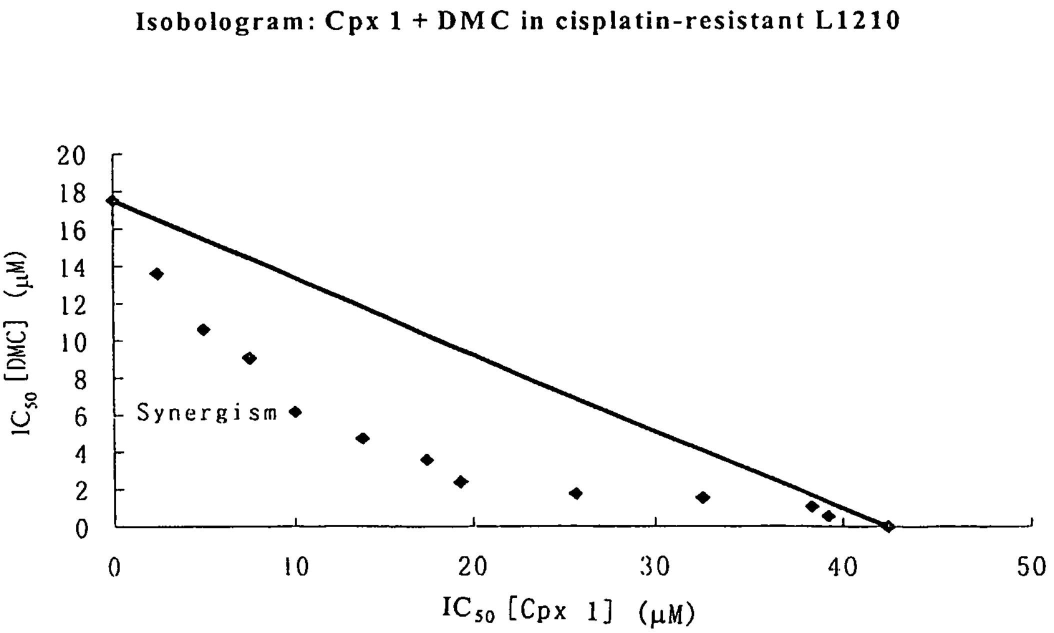
Isobologram: Cpx 1 + DMC in cisplatin-resistant L1210
20
18
16
14
12
10
8
6
4
2
0
IC50 [DMC] (μM)
Synergism
0
10
20
30
40
50
IC50 [Cpx 1] (μM)

Fig. 9

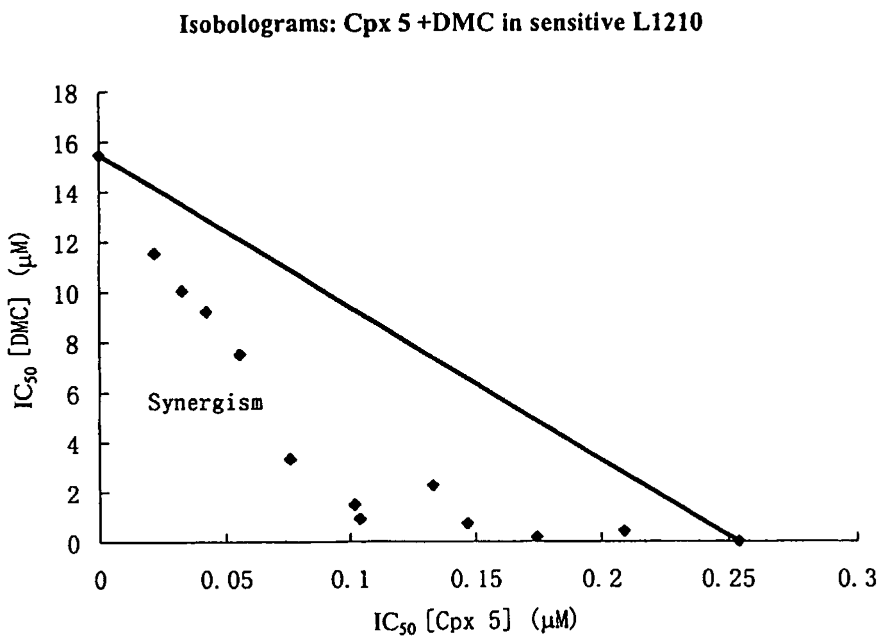
Isobolograms: Cpx 5 +DMC in sensitive L1210
18
16
14
12
10
8
6
4
2
0
$IC_{50}$ [DMC] (μM)
Synergism
0
0. 05
0. 1
0. 15
0. 2
0. 25
0. 3
$IC_{50}$ [Cpx 5] (μM)

Fig. 10

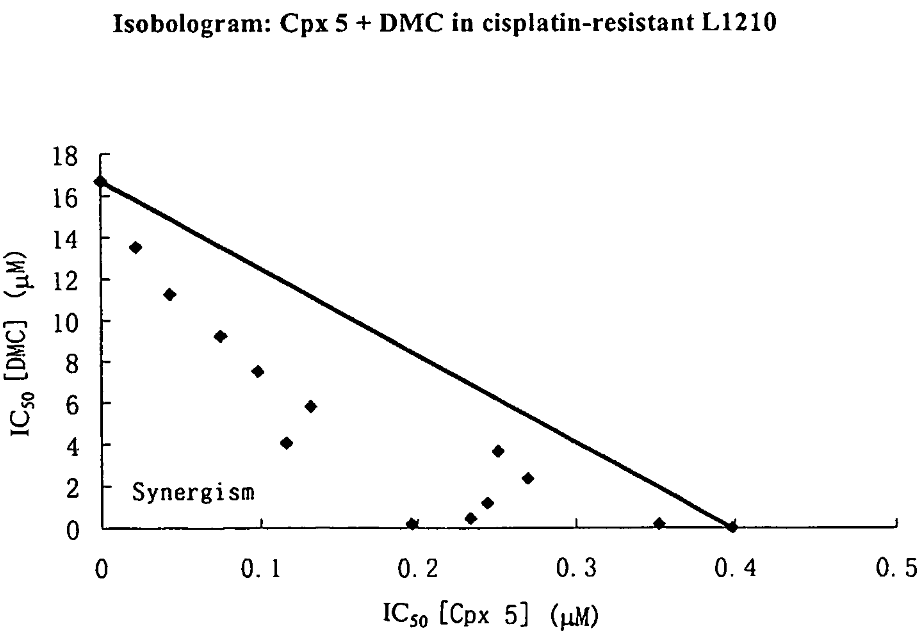
Isobologram: Cpx 5 + DMC in cisplatin-resistant L1210
18
16
14
12
10
8
6
4
2
0
$IC_{50}$ [DMC] (μM)
Synergism
0
0. 1
0. 2
0. 3
0. 4
0. 5
$IC_{50}$ [Cpx 5] (μM)

**Fig. 11**

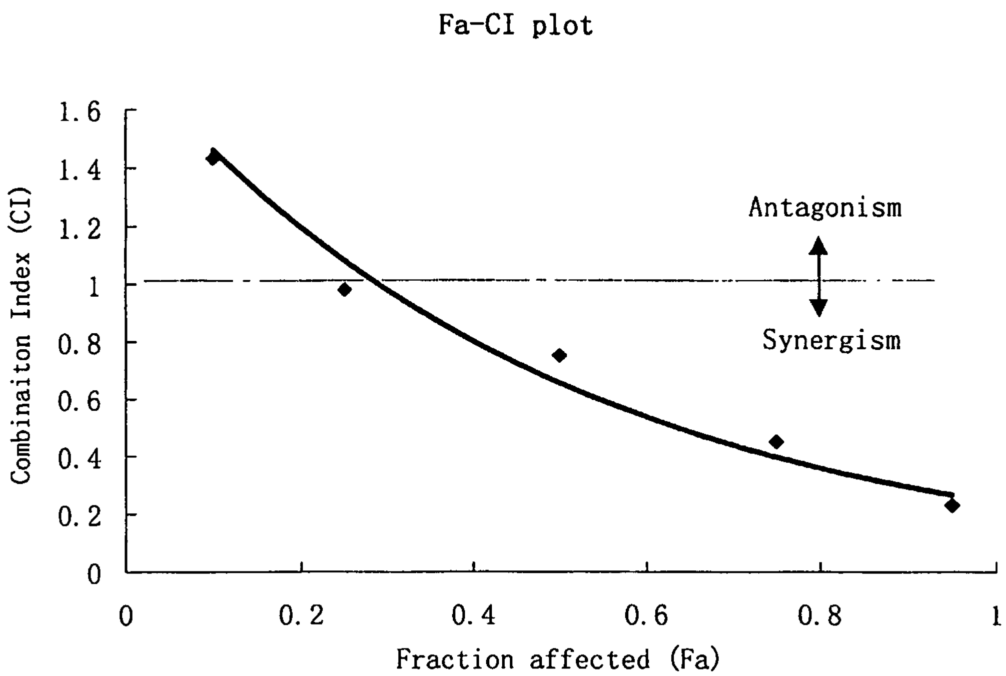
Fa-CI plot
Combinaiton Index (CI)
1.6
1.4
1.2
1
0.8
0.6
0.4
0.2
0
Antagonism
Synergism
0
0.2
0.4
0.6
0.8
1
Fraction affected (Fa)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6110907 A
- US 6110967 A
- WO 0004023 A1


### Non-patent literature cited in the description


- **ER-BIN YANG et al.** *Cancer Letters,* 1997, vol. 117, 93-98
- **CHOU TC ; TALALAY P.** A simple generalized equation for the analysis of multiple inhibitions of Michaelis-Menten kinetics systems. *JBiol Chem,* 1977, vol. 252, 6438-42
- **CHOU TC ; TALALAY P.** Generalized equations for the analysis of inhibitions of Michaelis-Menten and higher-order kinetics systems with two or more mutually exclusive and nonexclusive inhibitors. *Eur J Biochem,* 1981, vol. 115, 207-16
- **CHOU TC ; TALALAY P.** Quantitative analysis of dose-effect relationships: the combined effects of multiple drugs or enzyme inhibitors. *Adv Enzyme Regul,* 1984, vol. 22, 27-55
- Applications of the median-effect principle for the assessment of low-dose risk of carcinogens and for the quantitation of synergism and antagonism of chemotherapeutic agents. **CHOU TC ; TALALAY P.** In New Avenues in Development Cancer Chemotherapy, Bristol-Myers Symposium series. Academic Press, 1987, 37-64
- **CHOU TC.** Derivation and properties of Michaelis-Menton type and Hill type equations for reference ligands. *J Theor Biol,* 1976, vol. 59, 253-76
- The median-effect principle and the combination index for quantitation of synergism and antagonism, chap 2. **CHOU TC.** Synergism and Antagonism in Chemotherapy. Academic Press, 61-102
- **CHOU J ; CHOU TC.** Dose-effect analysis with microcomputers: quantitation of ED50, LD50, synergism, antagonism, low dose risk, receptor-ligand and enzyme kinetics. *EurJBiochem.,* 1987
- **CHOU J. ; CHOU T.-C.** Computerized simulation of dose reduction index (DRI) in synergistic drug combinations. *Pharmacologist,* 1988, vol. 30
- **BERMAN E. ; DUIGOU-OSTERNDORF R. ; KROWN S.E. ; FANUCCHI M.P. ; CHOU J. ; HIRSCH M.S. ; CLARKSON B.D. ; CHOU T.-C.** Synergistic cytotoxic effect of azidothymidine and recombinant interferon alpha on normal human bone marrow progenitor cells. *Blood,* 1989, vol. 74, 1281-6